# EUROPÄISCHE PATENTSCHRIFT

(11) **EP 1 791 814 B1**
(45) Veröffentlichungstag und Bekanntmachung des Hinweises auf die Patenterteilung: **29.06.2011**
(21) Anmeldenummer: 05785620.5
(22) Anmeldetag: 27.08.2005
(51) Int. Cl.: C07C 381/00

(54) **ORTHO-SUBSTITUIERTE PENTAFLUORSULFANYL-BENZOLE, VERFAHREN ZU IHRER HERSTELLUNG SOWIE IHRE VERWENDUNG ALS SYNTHESE-ZWISCHENSTUFEN**
ORTHO-SUBSTITUTED PENTAFLUOROSULFANYL BENZENES, METHOD FOR THE PRODUCTION THEREOF AND THEIR USE AS SYNTHESIS INTERMEDIATES
PENTAFLUORSULFANYL-BENZENES ORTHO-SUBSTITUES, PROCEDE POUR LES PREPARER ET LEUR UTILISATION EN TANT QU'INTERMEDIAIRES DE SYNTHESE

(30) Priorität: 11.09.2004 DE 102004043937
(43) Veröffentlichungstag der Anmeldung: 06.06.2007
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE); WECK, Remo, 65779 Kelkheim (DE)
(74) Vertreter: Michalski Hüttermann & Partner Patentanwälte
(86) Internationale Anmeldenummer: PCT/EP2005/009271
(87) Internationale Veröffentlichungsnummer: WO 2006/027129

(56) Entgegenhaltungen:
- WO-A-94/21606
- WO-A-94/22187
- DE-A1- 19 748 109
- SIPYAGIN A M ET AL: "Preparation of the first ortho-subsituted pentafluorosulfanylbenzenes" JOURNAL OF FLUORINE CHEMISTRY, ELSEVIER SEQUOIA. LAUSANNE, CH, Bd. 112, 2001, Seiten 287-295, XP002323728 ISSN: 0022-1139

## Beschreibung

Die Chemie der Pentafluorsulfanyl-Derivate hat in den letzten Jahren an Bedeutung zugenommen, insbesondere da neue Herstellungsverfahren gefunden wurden (Tetrahedron 56 (2000) 3399; Organic Letters 4(17) (2002) 3013). Allerdings sind bisher nur sehr wenige Verbindungen bekannt, die an einem Phenylring in ortho-Position zu der Pentafluorsulfanylgruppe andere Substituenten tragen als Wasserstoff und Fluor. Der einzige bekannte Syntheseweg (Journal of Fluorine Chemistry 112 (2001) 287) verwendet teure Reagentien wie AgF₂ und ist mit geringen Ausbeuten behaftet. Die Autoren begründen dies mit der großen Raumerfüllung der Pentafluorsulfanylgruppe, die ortho-Substitution generell sehr erschwert. Diese Meinung wird auch von anderen Autoren geteilt (J. Am. Chem. Soc. 84 (1962) 3064). Es war daher überraschend, das die elektrophile Substitution in ortho-Position zur Pentafluorsulfanylgruppe gelingt. Man erhält auf diesem neuen Weg neue ortho-substituierte Pentafluorsulfanyl-Benzole, die wertvolle Intermediate, beispielsweise zur Herstellung von Medikamenten, Diagnostika, Flüssigkristallen, Pestiziden, Herbiziden, Fungiziden, Nematiziden, Parasitiziden, Insektiziden, Akariziden, Arthropodiziden und Polymeren darstellen.

Die Erfindung betrifft ein Verfahren zur Herstellung der Pentafluorsulfanyl-Benzole der Formel I 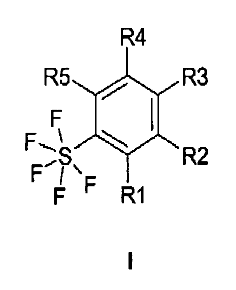 worin bedeuten
- R1: NO₂;
- R2: Wasserstoff oder Br;
einer der Reste R3 und R4 Wasserstoff oder Br;
und der andere der Reste R3 und R4
-O-Phenyl,
in dem der Phenylrest substituiert ist mit 3 oder 4 Resten ausgewählt aus der Gruppe bestehend aus Methyl, -SO₂CH₃, -COR13, wobei R13 OH oder Methoxy ist;
- R5: Wasserstoff;
oder deren Salze,
dadurch gekennzeichnet, dass man Verbindungen der Formel II durch elektrophile aromatische Substitution zu Verbindungen der Formel I umsetzt,

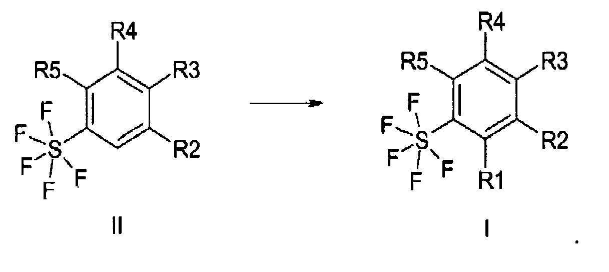

In einer weiteren Ausführungsform wird R2 in den Verbindungen der Formel I durch Wasserstoff beschrieben.

Die Erfindung betrifft ein Verfahren zur Herstellung der Verbindungen der Formel I oder deren Salze, dadurch gekennzeichnet, dass man Verbindungen der Formel II durch elektrophile aromatische Substitution zu Verbindungen der Formel I umsetzt, 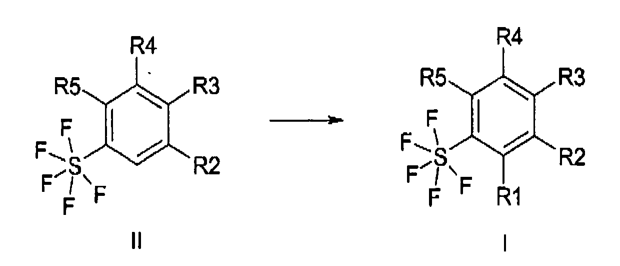 wobei R1 bis R5 die oben angegebene Bedeutung besitzen.

Bei der Herstellung der Verbindungen der Formel I geht man so vor, dass eine elektrophile aromatische Substitution durchgeführt wird, bevorzugt eine Nitrierung. In einer Ausführungsform wird nitriert (R1 = NO₂), wie beispielsweise in Houben-Weyl, Methoden der organischen Chemie, 4. Auflage, Organo-Stickstoff-Verbindungen IV, Teil 1, Georg Thieme Verlag Stuttgart 1992, S. 262-341 und in der dort zitierten Literatur beschrieben. Verbindungen der Formel II mit R4 = Phenyl , wobei das Phenyl wie unter R3 beschrieben substituiert sein kann, werden beispielsweise mit einer 90% wässrigen HNO₃-Lösung bei einer Temperatur zwischen -80°C und 20°C, bevorzugt zwischen -60°C und -20°C nitriert.

Aus den Verbindungen der Formel I mit R1 = NO₂ können die entsprechenden Aniline (R1 = NH₂) wie in R.C. Larock, Comprehensive Organic Transformations: A Guide to Functional Group Preparations, VCH Publishers, New York, Weinheim, 1999, 821-828 und der dort zitierten Literatur beschrieben hergestellt werden. Aus diesen Anilinen werden über die Diazoniumsalze nach dem Fachmann bekannter Methode, wie zum Beispiel in Houben-Weyl, Methoden der organischen Chemie, 4. Auflage, Organo-Stickstoff-Verbindungen I, Teil 2, Georg Thieme Verlag Stuttgart 1990, S. 1060-1136 sowie in den dort zitierten Literaturstellen beschrieben, die Verbindungen der Formel I mit weiteren Bedeutungen von R1 synthetisiert.

Die Ausgangsverbindungen der Formeln II sind käuflich erhältlich oder können nach analog zu in der Literatur beschriebenen und/oder dem Fachmann bekannten Verfahren hergestellt werden.

In den Ausgangsverbindungen können auch funktionelle Gruppen in geschützter Form oder in Form von Vorstufen vorliegen und dann in den nach dem oben beschriebenen Verfahren hergestellten Verbindungen der Formel I in die gewünschten Gruppen überführt werden. Entsprechende Schutzgruppentechniken sind dem Fachmann bekannt.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Beispiele für die vielfältigen Verwendungsmöglichkeiten von Pentafluorsulfanyl-Derivaten werden in den folgenden Veröffentlichungen beschrieben: WO9421606, WO03093228 A1 (Insektizide, Akarizide); DE19711953, GB2276379 (Herbizide); DE10124480, DE10353658, Angew. Chem. 1999, 111, 2174, Angew. Chem. 2000, 112, 4384 (Flüssigkristalle); WO03097591 (Medikamente, Diagnostika); US5220070, US5302692 (Polymere); WO03093228, WO9625401 (Pestizide), GB2276381, GB2276380 (Fungizide), US5637607 (Nematizide), W09947139 (Parasitizide), US6531501, WO9516676 (Arthropodizide).

Die Verbindungen der Formel I können in Form ihrer Salze isoliert werden. Diese werden nach den üblichen Methoden durch Umsetzung mit Säuren oder Basen erhalten. Als Säureadditionssalze kommen dabei beispielsweise Halogenide, insbesondere Hydrochloride, Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate und Trifluoracetate in Frage, im Fall der Herstellung von Wirkstoffen bevorzugt pharmazeutisch verträgliche Salze. Enthalten die Verbindungen eine Säuregruppe, können sie Salze mit Basen bilden, beispielsweise Alkalimetallsalze, vorzugsweise Natrium- oder Kaliumsalze, oder Ammoniumsalze, zum Beispiel als Salze mit Ammoniak oder organischen Aminen oder Aminosäuren. Sie können auch als Zwitterion vorliegen.

### Liste der Abkürzungen:

- DIP: Diisopropylether
- DIPEA: Diisopropylethylamin
- DME: 1,2-Dimethoxyethan
- DMF: N,N-Dimethylformamid
- EE: Ethylacetat
- eq.: Äquivalent
- HEP: n-Heptan
- HOAc: Essigsäure
- MeOH: Methanol
- mp: Schmelzpunkt
- MTB: tert.-Butyl-methylether
- NBS: 2-Brom-isoindol-1,3-dion
- NCIS: 2-Chlor-isoindol-1,3-dion
- RT: Raumtemperatur
- THF: Tetrahydrofuran

### Beispiel 1: 5-Methansulfonyl-2-methyl-4-(4-nitro-3-pentafluorsulfanyl-phenoxy)-benzoesäure-methylester

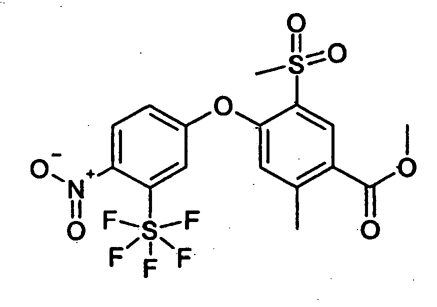

### a) 3-Pentafluorsulfanyl-phenol

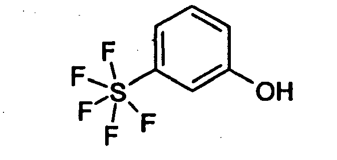

5.0 g 3-Pentafluorsulfanyl-anilin (Tetrahedron 56, (2000) 3399) wurden in 50 ml einer 35% wäßrigen H₂SO₄-Lösung suspendiert. Bei 0°C wurde dann eine Lösung von 1.57 g NaNO₂ in 5 ml Wasser innerhalb 10 Minuten zugetropft. 40 Minuten lang wurde bei 0°C nachgerührt. Zu dieser Suspension wurde dann eine auf 0°C gekühlte Lösung von 8.56 g Cu(NO₃)₂ in 50 ml Wasser zugegeben. Direkt danach wurden noch 3.26 g Cu₂O zugegeben, wobei deutliche Gasentwicklung zu beobachten war. 3 mal wurde mit je 100 ml CH₂Cl₂ extrahiert, die org. Phase mit 100 ml einer gesättigten wäßrigen NaCl-Lsg. gewaschen, über MgSO₄ getrocknet und das Solvens im Vakuum entfernt. Chromatographie an Kieselgel mit DIP lieferte 3.5 g des Phenols als farbloses Öl.
R_{f} (EE/HEP 1:10) = 0.15

### b) 5-Methansulfonyl-2-methyl-4-(3-pentafluorsulfanyl-phenoxy)-benzoesäure-methylester

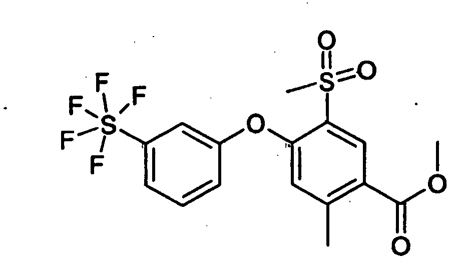

5.00 g 3-Pentafluorsulfanyl-phenol, 6.98 g 4-Brom-5-methansulfonyl-2-methyl-benzoesäure-methylester (Journal of Medicinal Chemistry (1997), 40(13), 2017) und 22.20 g Cs₂CO₃ wurden in 200 ml wasserfreiem DMF 2 h bei 100°C gerührt. Man ließ abkühlen, das Reaktionsgemisch wurde auf 1 I Wasser gegossen und 16 h bei RT gerührt. Dann wurde das Produkt abfiltriert und im Vakuum getrocknet. Man erhielt 8.20 g blassgelber Kristalle, mp 139°C (unter Zersetzung).

| | |
|---|---|
| R_{f} (DIP) = 0.27 | MS (ES⁺): 446 |

### c) 5-Methansulfonyl-4-(4-nitro-3-pentafluorsulfanyl-phenoxy)-2-methyl-benzoesäure-methylesters

3.50 g 5-Methansulfonyl-2-methyl-4-(3-pentafluorsulfanyl-phenoxy)-benzoesäure-methylester wurde bei -40°C in 100 ml 90% HNO₃ gelöst. 10 Minuten wurde bei dieser Temperatur gerührt und dann das Reaktionsgemisch auf 800 g Eis gegossen. Dieses Gemisch wurde 10 Minuten gerührt und anschließend das Produkt abgesaugt. Man erhielt 3.89 g eines blassgelben Feststoffs, mp145-148°C (unter Zersetzung).
R_{f} (DIP) = 0.09

### Beispiel 2 (nicht Teil der Erfindung): 4-(4-Amino-3-pentafluorsulfanyl-phenoxy)-5-methansulfonyl-2-methyl-benzoesäure-methylester

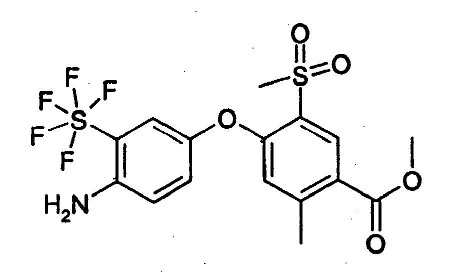

3.80 g 5-Methansulfonyl-4-(4-nitro-3-pentafluorsulfanyl-phenoxy)-2-methyl-benzoesäure-methylester (Beispiel 1) wurden in 30 ml HOAc sowie 30 ml MeOH gelöst, mit 200 mg Pd/C (10%) versetzt und 24 h bei 6 bar Wasserstoffdruck hydriert. Da die Umsetzung noch nicht vollständig war, wurden weitere 300 mg Pd/C (10%), 30 ml HOAc und 30 ml MeOH zugegeben und weitere 24 h bei 6 bar Wasserstoffdruck hydriert. Anschließend wurde der Katalysator abfiltriert und die Solventien im Vakuum entfernt. Man erhielt 3.4 g eines hellgrauen Feststoffs, mp 175°C (unter Zersetzung).
Rf (MTB) = 0.44

### Beispiel 3 (nicht Teil der Erfindung): 4-(4-Chlorsulfonyl-3-pentafluorsulfanyl-phenoxy)-5-methansulfonyl-2-methyl-benzoesäure-methylester

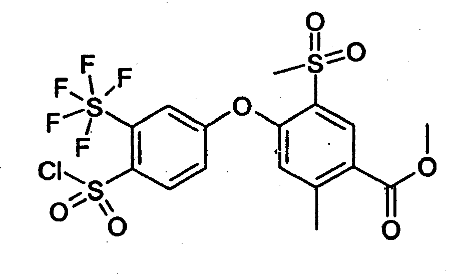

3.4 g 4-(4-Amino-3-pentafluorsulfanyl-phenoxy)-5-methansulfonyl-2-methyl-benzoesäure-methylester (Beispiel 2) wurden in 30 ml HOAc gelöst, mit 30 g Eis und anschließend mit 30 ml einer gesättigten wäßrigen HCl-Lösung versetzt. Zu dieser Lösung wurde bei 0°C eine Lösung von 0.56 g NaNO₂ in 5 ml Wasser innerhalb von 5 Minuten zugetropft. 10 Minuten wurde bei 0°C gerührt. Diese Lösung wurde dann portionsweise zu einer 0°C kalten Suspension aus 12.4 mg CuCl und 125.6 mg CuCl₂ (Dihydrat) in 100 ml einer mit SO₂ gesättigten Essigsäure addiert. 2 h wurde bei RT gerührt, dann mit 300 ml Wasser verdünnt und 3 mal mit je 200 ml EE extrahiert. Über MgSO₄ wurde getrocknet und das Solvens im Vakuum entfernt. Man erhielt 3.5 g eines viskosen Öls, das ohne Reinigung weiter umgesetzt wurde.

### Beispiel 4 (nicht Teil der Erfindung): Natrium-4-(2-methansulfonyl-4-methoxycarbonyl-5-methyl-phenoxy)-2-pentafluorsulfanyl-benzolsulfinat

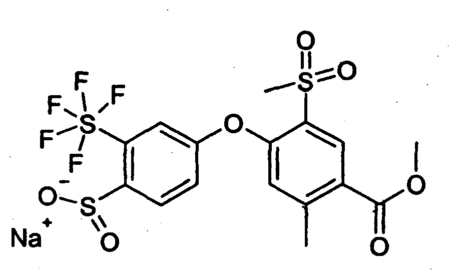

3.5 g 4-(4-Chlorsulfonyl-3-pentafluorsulfanyl-phenoxy)-5-methansulfonyl-2-methyl-benzoesäure-methylester (Beispiel 3) wurden portionsweise zu einer 70°C warmen Lösung von 8.10 g Na₂SO₃ in 75 ml Wasser addiert und dabei mit 10 molarer wässriger NaOH-Lösung der pH bei etwa pH=10 gehalten. Anschließend wurde 45 Minuten bei 70°C nachgerührt, dann ließ man abkühlen und stellte mit wäßriger HCl-Lösung den pH auf pH=2. 3 mal wurde mit je 200 ml EE extrahiert. Über MgSO₄ wurde getrocknet und das Solvens im Vakuum entfernt. Der Rückstand wurde in 100 ml Wasser suspendiert, mit einer 2 molaren wäßrigen NaOH-Lösung auf pH=10 gestellt und die flüchtigen Bestandteile im Vakuum entfernt. Anschließend wurde zunächst 2 mal mit je 100 ml Toluol, dann mit 100 ml wasserfreiem DMF co-evaporiert und der Rückstand (3.0 g) ohne Reinigung weiter umgesetzt.

### Beispiel 5 (nicht Teil der Erfindung): 5-Methansulfonyl-4-(4-methansulfonyl-3-pentafluorsulfanyl-phenoxy)-2-methyl-benzoesäure-methylester

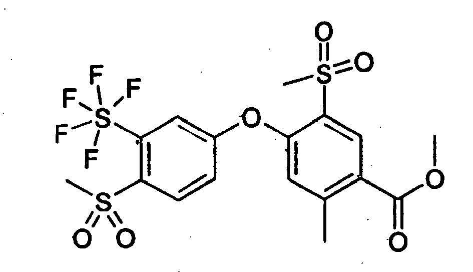

und 5-Methansulfonyl-4-(4-methoxysulfinyl-3-pentafluorsulfanyl-phenoxy)-2-methyl-benzoesäure-methylester

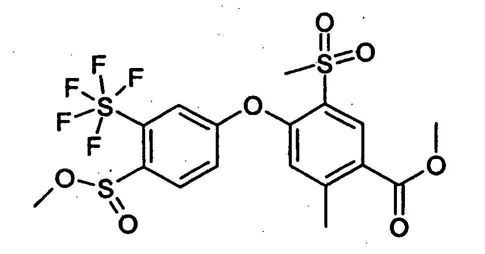

3.0 g Natrium-4-(2-methansulfonyl-4-methoxycarbonyl-5-methyl-phenoxy)-2-pentafluorsulfanyl-benzolsulfinat (Beispiel 4) wurden in 100 ml wasserfreiem DMF gelöst, 4.0 g CH₃l hinzugegeben und 9 h bei 45°C gerührt. Anschließend wurde das Reaktionsgemisch 2 Tage bei RT stehen gelassen. Dann wurde das Solvens im Vakuum entfernt und der Rückstand mit 100 ml Wasser sowie 100 ml EE aufgenommen. Dann wurden 50 ml einer 5% wäßrigen NaHSO₄-Lösung zugegeben und die Phasen getrennt. Anschließend wurde 3 mal mit je 100 ml EE extrahiert. Über MgSO₄ wurde getrocknet, das Solvens im Vakuum entfernt und der Rückstand an Kieselgel mit MTB/DIP 1:1 chromatographiert. Man erhielt 0.59 g 5-Methansulfonyl-4-(4-methansulfonyl-3-pentafluorsulfanyl-phenoxy)-2-methyl-benzoesäure-methylester und 0.49 g 5-Methansulfonyl-4-(4-methoxysulfinyl-3-pentafluorsulfanyl-phenoxy)-2-methyl-benzoesäure-methylester.
Rf (MTB/DIP 1:1) = 0.13: 5-Methansulfonyl-4-(4-methansulfonyl-3-pentafluorsulfanyl-phenoxy)-2-methyl-benzoesäure-methylester
Rf (MTB/DIP 1:1) = 0.32: 5-Methansulfonyl-4-(4-methoxysulfinyl-3-pentafluorsulfanyl-phenoxy)-2-methyl-benzoesäure-methylester

### Beispiel 6: 3-Brom-4-(2,6-dibrom-3-nitro-4-pentafluorsulfanyl-phenoxy)-5-methansulfonyl-2-methyl-nitrobenzol

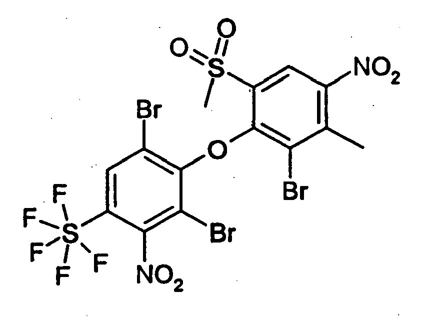

### a) 4-Pentafluorsulfanyl-phenol

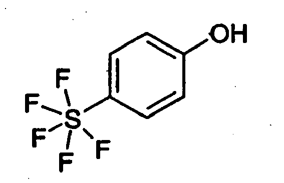

40.00 g 4-Pentafluorsulfanyl-anilin (Tetrahedron 56, (2000) 3399) wurden in 500 ml einer 35% wäßrigen H₂SO₄-Lösung suspendiert. Bei 0°C wurde dann eine Lösung von 13.85 g NaNO₂ in 30 ml Wasser innerhalb 10 Minuten zugetropft. 35 Minuten lang wurde bei 0°C nachgerührt. Zu dieser Suspension wurde dann eine auf 0°C gekühlte Lösung von 171.10 g Cu(NO₃)₂ in 200 ml Wasser zugegeben. Direkt danach wurde noch 26.11g Cu₂O zugegeben, wobei deutliche Gasentwicklung zu beobachten war. 2 Stunden wurde beim RT nachgerührt, dann wurde 3 mal mit je 200 ml CH₂Cl₂ extrahiert, die vereinigten organischen Phasen über MgSO₄ getrocknet und das Solvens im Vakuum entfernt. Chromatographie an Kieselgel mit DIP lieferte 3.5 g des Phenols als farbloses Öl.

| | |
|---|---|
| R_{f} (EE/HEP 1:10) = 0.15 | MS (EI) : 220 |

### b) 5-Methansulfonyl-2-methyl-4-(4-pentafluorsulfanyl-phenoxy)-benzoesäure-methylester

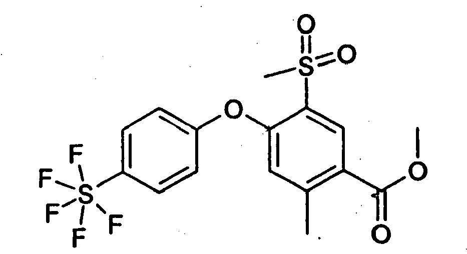

5.00 g 4-Pentafluorsulfanyl-phenol, 6.98 g 4-Brom-5-methansulfonyl-2-methyl-benzoesäure-methylester (Journal of Medicinal Chemistry (1997), 40(13), 2017) und 22.20 g Cs₂CO₃ wurden in 200 ml wasserfreiem DMF 2 h bei 100°C gerührt. Man ließ abkühlen, das Reaktionsgemisch wurde auf 1 I Wasser gegossen und der Feststoff abgesaugt und getrocknet. Man erhielt 8.00 g eines amorphen Feststoffs.

| | |
|---|---|
| R_{f} (EE/HEP 1:10) = 0.26 | MS (ES⁺): 446 |

### c) 3-Brom-4-(2,6-dibrom-4-pentafluorsulfanyl-phenoxy)-5-methansulfonyl-2-methyl-benzoesäure-methylester

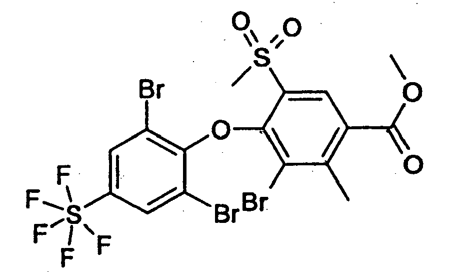

8.00 g 5-Methansulfonyl-2-methyl-4-(4-pentafluorsulfanyl-phenoxy)-benzoesäure-methylester wurden in 200 ml Trifluoressigsäure gelöst und 20 ml 97% H₂SO₄. zugegeben. Bei RT wurden dann 9.57 g NBS in 3 Portionen zugegeben. 2 Stunden wurde bei RT gerührt, anschließend weitere 4.00 g NBS in zwei Portionen zugegeben. Dann wurde 16 Stunden bei RT stehen gelassen, dann auf 300 g Eis gegossen und mit gesättigter wäßriger Na₂CO₃-Lösung auf pH > 7 gestellt. Dann wurden 5.00 g Na₂SO₃ addiert und 3 mal mit je 500 ml EE extrahiert. 3 mal wurde mit halbgesättigter wäßriger Na₂CO₃-Lösung gewaschen, anschließend über MgSO₄ getrocknet und das Solvens im Vakuum entfernt. Man erhielt 6.4 g eines amorphen Feststoffs.
R_{f} (DIP) = 0.48

### d) 3-Brom-4-(2,6-dibrom-4-pentafluorsulfanylphenoxy)-5-methansulfonyl-2-methyl-benzoesäure

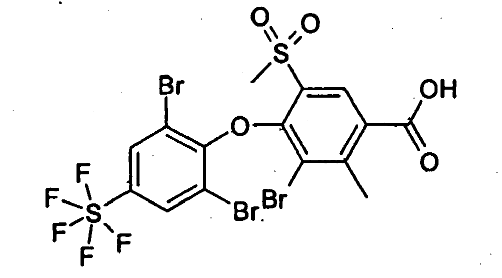

6.2 g 3-Brom-4-(2,6-dibrom-4-pentafluorsulfanyl-phenoxy)-5-methansulfonyl-2-methyl-benzoesäure-methylester wurden in 300 ml MeOH gelöst und 100 ml Wasser sowie 5.45 ml einer 2 molaren wäßrigen NaOH-Lösung zugegeben. 20 Stunden wurde bei RT gerührt, anschließend 3 Stunden zum Rückfluss erhitzt. Das MeOH wurde im Vakuum entfernt, mit 200 ml Wasser verdünnt und mit einer wäßrigen HCl-Lösung auf pH < 2 gestellt. 10 Minuten wurde bei RT nachgerührt und dann das Produkt abgesaugt. Man erhielt 6.0 g eines farblosen Feststoffs, mp 228°C (unter Zersetzung).

| | |
|---|---|
| R_{f} (DIP 2%HOAc) = 0.15 | MS (ES⁻) : 666 |

### e) 3-Brom-4-(2,6-dibrom-3-nitro-4-pentafluorsulfanyl-phenoxy)-5-methansulfonyl-2-methyl-nitrobenzol

200 mg 3-Brom-4-(2,6-dibrom-4-pentafluorsulfanyl-phenoxy)-5-methansulfonyl-2-methyl-benzoesäure wurden in 500 µl einer 90% wäßrigen HNO₃-Lösung und 50 µl 97% H₂SO₄ eine Stunde bei RT gerührt. Dann wurde auf 50 ml Wasser gegossen, das Produkt abgesaugt und im Vakuum getrocknet. Man erhielt 200 mg eines

| | |
|---|---|
| amorphen Feststoffs. | MS (ES⁻) : 711 |

## Patentansprüche

1. Verfahren zur Herstellung der Verbindungen der Formel I 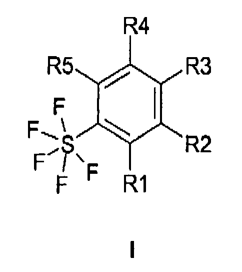 worin bedeuten
R1 NO₂;
R2 Wasserstoff oder Br;
einer der Reste R3 und R4 Wasserstoff oder Br;
und der andere der Reste R3 und R4 -O-Phenyl,
in dem der Phenylrest substituiert ist mit 3 oder 4 Resten ausgewählt aus der Gruppe bestehend aus Methyl, -SO₂CH₃, -COR13, wobei R13 OH oder Methoxy ist;
R5 Wasserstoff;
oder deren Salze,
**dadurch gekennzeichnet, dass** man Verbindungen der Formel II durch elektrophile aromatische Substitution zu Verbindungen der Formel I umsetzt,

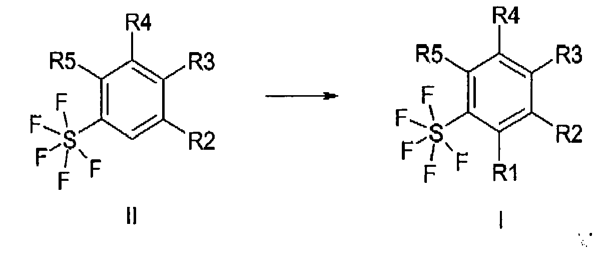

## Claims

1. Process for preparing the compounds of the formula I 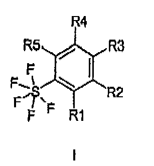 in which
R1 is NO₂;
R2 is hydrogen or Br;
one of the R3 and R4 radicals is hydrogen or Br;
and the other of the R3 and R4 radicals is -O-phenyl, in which the phenyl radical is substituted by 3 or 4 radicals selected from the group consisting of methyl, -SO₂CH₃, -COR13 where R13 is OH or methoxy;
R5 is hydrogen;
or salts thereof,
**characterized in that** compounds of the formula II are converted by electrophilic aromatic substitution to compounds of the formula I

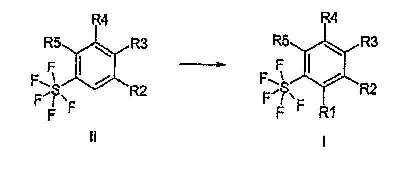

## Revendications

1. Procédé pour la préparation des composés de formule I 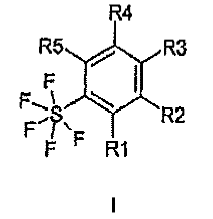 dans laquelle
R1 représente NO₂;
R2 représente un atome d'hydrogène ou Br ;
l'un des radicaux R3 et R4 représente un atome d'hydrogène ou Br ;
et l'autre des radicaux R3 et R4 représente un groupe -O-phényle, dans lequel le radical phényle est substitué par 3 ou 4 radicaux choisis dans l'ensemble constitué par les groupes méthyle, -SO₂CH₃, -COR13, R13 représentant OH ou le groupe méthoxy ;
R5 représente un atome d'hydrogène ;
ou leurs sels,
**caractérisé en ce qu'**on convertit par substitution aromatique électrophile des composés de formule II en composés de formule I
